# EUROPEAN PATENT SPECIFICATION

(11) **EP 2 870 488 B1**
(45) Date of publication and mention of the grant of the patent: **15.02.2017**
(21) Application number: 13763116.4
(22) Date of filing: 28.06.2013
(51) Int. Cl.: G01R 33/54, G01R 33/565, A61B 5/055, A61B 5/00

(54) **A METHOD FOR MAINTAINING GEOMETRIC ALIGNMENT OF MR SCANS IN CASES OF STRONG PATIENT MOTION**
VERFAHREN ZUR BEWAHRUNG DER GEOMETRISCHEN AUSRICHTUNG VON MRT-SCANS BEI STARKER BEWEGUNG DES PATIENTEN
PROCÉDÉ DE MAINTIEN DE L'ALIGNEMENT GÉOMÉTRIQUE D'IMAGES PAR IRM EN CAS DE MOUVEMENTS IMPORTANTS DU PATIENT

(30) Priority: 05.07.2012 US 201261668155 P
(43) Date of publication of application: 13.05.2015
(73) Proprietor: Koninklijke Philips N.V., 5656 AE Eindhoven (NL); Philips GmbH, 20099 Hamburg (DE)
(72) Inventor: SENEGAS, Julien, NL-5656 AE Eindhoven (NL); KOKEN, Peter, NL-5656 AE Eindhoven (NL); VIK, Torbjørn, NL-5656 AE Eindhoven (NL)
(74) Representative: Steffen, Thomas
(86) International application number: PCT/IB2013/055324
(87) International publication number: WO 2014/006550

(56) References cited:
- US-A1- 2010 130 849
- US-B1- 6 275 721
- US-B1- 6 898 302
- T. ERNST ET AL: "Simultaneous correction for interscan patient motion and geometric distortions in echoplanar imaging", MAGNETIC RESONANCE IN MEDICINE, vol. 42, no. 1, 1 July 1999 (1999-07-01), pages 201-205, XP055091975, ISSN: 0740-3194, DOI: 10.1002/(SICI)1522-2594(199907)42:1<201::A ID-MRM27>3.0.CO;2-Y
- DERBYSHIRE J A ET AL: "DYNAMIC SCAN-PLANE TRACKING USING MR POSITION MONITORING", JOURNAL OF MAGNETIC RESONANCE IMAGING, SOCIETY FOR MAGNETIC RESONANCE IMAGING, OAK BROOK, IL, US, vol. 8, no. 4, 1 July 1998 (1998-07-01), pages 924-932, XP008018580, ISSN: 1053-1807

## Description

The present application relates generally to magnetic resonance (MR) imaging. It finds particular application in conjunction with maintaining geometric alignment of scans in cases of strong patient motion and will be described with particular reference thereto. However, it is to be understood that it also finds application in other usage scenarios and is not necessarily limited to the aforementioned application.

An MR examination generally includes of a list of scans with different MR contrasts and/or different orientations. For the reading radiologist, it is often desirable to compare these different scans slice by slice, for example, to analyze the appearance of a lesion under different MR contrast types. If the position of the patient changes between two consecutive scans, the same voxel in the scans before and after motion will point to different locations within the body of the patient. This hampers slice-by-slice comparison of these scans and makes diagnosis more difficult and less reliable. Further, post-processing applications, such as subtraction of pre-contrast and post-contrast scans, may also be prevented from being performed.

Deviations between scans are particularly severe when the patient is repositioned by the operator during examination. Deviations between scans may also be severe when the patient feels uncomfortable in the scanner bore, the technical assistant has forgotten to put a sensor (e.g., a breathing bell) that is required for a particular scan, or there is a technical problem with, for example, a coil.

When an examination has been interrupted due to repositioning of the patient, it is currently difficult to resume the examination while maintaining the geometric alignment of the scans before and after repositioning. The technical assistant may decide, in some cases, to repeat the whole examination, which leads to time delays and increased time pressure. Alternatively, after the completion of the examination, it is also possible to register the reconstructed images and realign the images acquired before and after repositioning. However, in this case, the image quality of the registered scans is lower due to increased partial volume effects.

In examinations that use automated scan planning software, it is possible to mitigate the effects of repositioning by re-launching the automated scan planning functionality. Generally, this will require the acquisition of a new survey scan as a basis for the planning. Re-launching the automated scan planning functionality advantageously ensures alignment between the initial and the repeated survey scan. However, it does not necessarily ensure alignment between the diagnostic scans acquired before and after repositioning.

US 2010/130849 A1 relates to a method for motion compensation including acquiring an initial volumetric localizer to establish an initial object position and initial object orientation at an initial state, acquiring a fast localizer of the object at a present state, aligning the fast localizer to the initial volumetric localizers to determine object motion between the initial state and the present state, and modifying an imaging protocol using the object position and orientation at the present state.

The article by Ernst T. et al.: "Simultaneous correction for interscan patient motion and geometric distortions in echoplanar imaging", Magnetic Resonance in Medicine, vol. 42, no. 1, July 1, 1999, pages 201-205, presents a method for simultaneous correction of linear geometric distortions and interscan patient motion in echoplanar imaging (EPI). The method is based on a generalized surface-based coregistration algorithm, which accounts for a complete 3-dimensional affine transformation, i.e., rotations, translations, scaling, and shearing, between two volumetric image data sets. Any minimally distorted high-resolution scan may serve as a reference data set, to which the EPI data set is matched.

The article by Derbyshire J. A. et al.: "Dynamic scan-plane tracking using MR position monitoring", Journal of Magnetic Resonance Imaging, vol. 8, no. 4, July 1, 1998, pages 924-932, presents an MR-based method for tracking subject motion. The technique identifies subject motion from the three-dimensional positions of three small samples attached to the subject in a fixed, triangular configuration. The updated positions of these samples relative to their initial positions determine a rigid body transformation. Applied to the MRI scan prescription via adaptive feedback controls, this transformation yields an updated MRI scan plane that tracks the prescribed imaging section as the subject moves.

The present application provides a new and improved system and method which overcome the above-referenced problems and others.

In accordance with one aspect, a magnetic resonance (MR) system for maintaining geometric alignment of diagnostic scans during an examination of a patient is provided in independent claim 1.

In accordance with another aspect, a magnetic resonance (MR) method for maintaining geometric alignment of diagnostic scans during an examination of a patient is provided in independent claim 11.

One advantage resides in geometric alignment of scans before and after patient repositioning.

Another advantage resides in geometrically aligned scans acquired before and after patient repositioning that do no exhibit partial volume effects.

Another advantage resides in geometrically aligned scans before and after patient repositioning without loss of image quality.

Another advantage resides in geometrically aligned scans notwithstanding strong patient motion.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 illustrates a magnetic resonance (MR) system for geometric alignment between scans.
FIGURE 2 illustrates a method for geometrically aligning a diagnostic scan performed after patient motion to a diagnostic scan performed before the patient motion.
FIGURE 3 illustrates an example of an examination with patient motion.

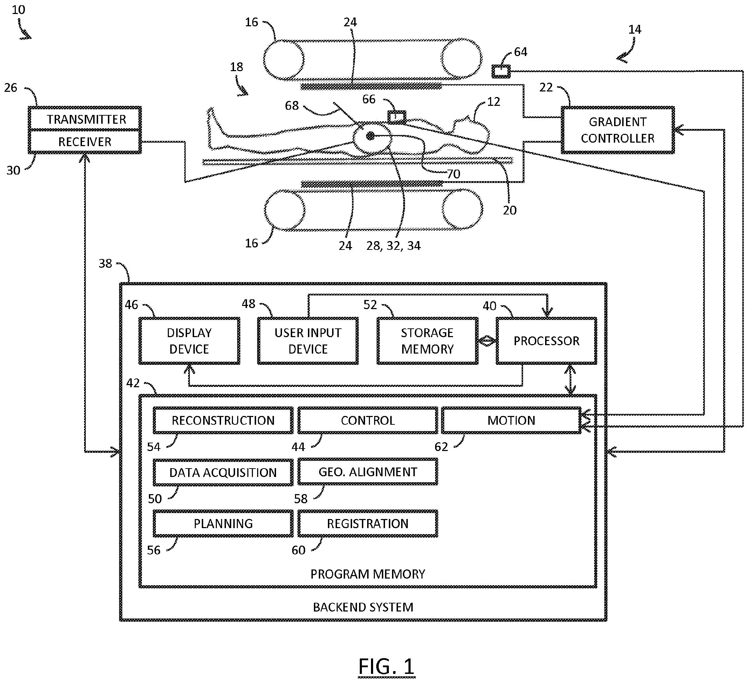

With reference to FIGURE 1, a magnetic resonance (MR) system **10** utilizes MR to perform an examination of a patient **12.** The MR system **10** includes a scanner **14.** The scanner **14** includes a main magnet **16** that creates a strong, static B₀ magnetic field extending through an examination volume **18.** The examination volume **18** is sized to accommodate the patient **12,** which is positioned in the examination volume **18** during the examination. A patient support **20** supports the patient **12** in the scanner **14** and facilitates positioning the patient **12** in the examination volume **18.**

The main magnet **16** typically employs superconducting coils to create the static B₀ magnetic field. However, the main magnet **16** can also employ permanent or resistive magnets. Insofar as superconducting coils are employed, the main magnet **16** includes a cooling system, such as a liquid helium cooled cryostat, for the superconducting coils. The strength of the static B₀ magnetic field is commonly one of 0.23 Tesla, 0.5 Tesla, 1.5 Tesla, 3 Tesla, 7 Tesla, and so on in the examination volume **18,** but other strengths are contemplated.

As illustrated, the main magnet **16** is an open type and includes two superconducting coils spaced apart to define the examination volume **18.** The superconducting coils produce the static B₀ magnetic field similar to the way in which a Helmholtz coil would. The advantage of an open magnet is that it provides easy access to the patient **12.** However, different types of main magnets can also be employed. For example, a split cylindrical main magnet and/or a cylindrical main magnet can be employed. A split cylindrical main magnet is similar to a cylindrical main magnet, which includes a cryostat, except that the cryostat is split into two sections to allow access to the iso-plane of the magnet.

A gradient controller **22** of the scanner **14** is controlled to superimpose magnetic field gradients, such as x, y and z gradients, on the static B₀ magnetic field in the examination volume **18** using a plurality of magnetic field gradient coils **24** of the scanner. The magnetic field gradients spatially encode magnetic spins within the examination volume **18.** Typically, the plurality of magnetic field gradient coils **24** include three separate magnetic field gradient coils spatially encoding in three orthogonal spatial directions.

Further, one or more transmitters **26,** such as a transceiver, of the scanner **14** are controlled to transmit B₁ resonance excitation and manipulation radio frequency (RF) pulses into the examination volume **18** with one or more transmit coils **28,** such as a whole body coil and/or a surface coil, of the scanner **14.** The B₁ pulses are typically of short duration and, when taken together with the magnetic field gradients, achieve a selected manipulation of magnetic resonance. For example, the B₁ pulses excite the hydrogen dipoles to resonance and the magnetic field gradients encode spatial information in the frequency and phase of the resonance signal. By adjusting the RF frequencies, resonance can be excited in other dipoles, such as phosphorous, which tend to concentrate in known tissues, such as bones.

One or more receivers **30,** such as a transceiver, of the scanner **14** are controlled to receive spatially encoded magnetic resonance signals from the examination volume **18** and demodulate the received spatially encoded magnetic resonance signals to MR data sets. The MR data sets include, for example, k-space data trajectories. To receive the spatially encoded magnetic resonance signals, the receivers **30** use one or more receive coils **32,** such as a whole body coil and/or a surface coil, of the scanner **14.** The receivers **30** typically store the MR data sets in a buffer memory.

As illustrated, the transmit coils **28** and the receive coils **32** include a surface coil **34** positioned on the surface of the subject **12.** The surface coil **34** is employed as both a transmit coil and a receive coil. However, it is to be appreciated that the surface coil **34** can be employed as only one of a transmit coil and a receive coil.

A backend system **38** coordinates the examination of the patient **12.** The examination includes performing a plurality of diagnostic scans of the patient **12,** typically with different contrasts and/or different orientations. The backend system **38** includes at least one processor **40** and at least one program memory **42.** The program memory **42** includes processor executable instructions that, when executed by the processor **40,** coordinate the examination of the patient **12.** The processor **40** executes the processor executable instructions to coordinate the examination of the patient **12.**

A control module **44** of the processor executable instructions controls overall operation of the backend system **38.** The control module **44** suitably displays a graphical user interface (GUI) to an operator of the backend system **38** using a display device **46** of the backend system **38.** Further, the control module **44** suitably allows the operator to interact with the GUI using a user input device **48** of the backend system **38.** For example, the operator can interact with the GUI to instruct the backend system **38** to coordinate the examination of the patient **12.**

A data acquisition module **50** of the processor executable instructions performs the diagnostic scans of the patient **12.** For each diagnostic scan, the data acquisition module **50** receives a scan plan. The scan plan includes one or more scan parameters, such as number of slices, and one or more imaging sequences. An imaging sequence defines a sequence of B₁ pulses and/or magnetic field gradients that produce spatially encoded MR signals from the examination volume **18.** The scan plan defines the geometry of the diagnostic scan, such as slice positioning. The data acquisition module **50** then controls the transmitters **26** and/or the gradient controller **22** according to the scan parameters to implement the imaging sequence within the examination volume **18.** Further, the data acquisition module **50** controls the receivers **30** according to the scan parameters to capture the spatially encoded MR signals to an MR data set. The MR data set is typically stored in at least one storage memory **52** of the backend system **38.**

A reconstruction module **54** of the processor executable instructions reconstructs the MR data sets of the diagnostic scans into MR images or maps of the subject **12.** This includes, for each MR signal captured by the MR data sets, spatially decoding the spatial encoding by the magnetic field gradients to ascertain a property of the MR signal from each spatial region, such as a pixel or voxel. The intensity or magnitude of the MR signal is commonly ascertained, but other properties related to phase, relaxation time, magnetization transfer, and the like can also be ascertained. The MR images or maps are typically stored in the storage memory **52.**

A planning module **56** of the processor executable instructions plans the diagnostic scans. This includes performing an initial survey scan, such as a fast, three-dimensional (3D) survey scan, of the patient **12** using the data acquisition module **50.** Based on the initial survey scan, the planning module **56** determines the scan plan, including the scan parameters and the imaging sequences, for each of the diagnostic scans. Determining the scan plan for the diagnostic scan includes determining the geometry of the diagnostic scan. The geometry of the diagnostic scan can be determined automatically and/or manually with reference to the coordinate frame of the initial survey scan.

To manually determine the geometry of the diagnostic scan, the initial survey scan is displayed on the GUI with a scan geometry overlaid thereon. The scan geometry can be a default scan geometry or determined automatically, as described below. Further, the scan geometry can, for example, be represented by the positions of slices. An operator of the system then manipulates the scan geometry using the user input device **48** as desired. Manipulation includes, for example, positioning and/or sizing.

To automatically determine the geometry of the diagnostic scan, the planning module **56** uses an examination template specifying the geometries of the diagnostic scans in relation to patient landmarks. The planning module **56** analyzes the initial survey scan, within image-space or k-space, to locate the patient landmarks and then, based on the examination template, determines the geometries of the diagnostic scans relative to the landmarks. The examination template is, for example, stored in the storage memory **52** and generated prior to beginning the examination.

A geometric alignment module **58** of the processor executable instructions, in response to patient motion between the diagnostic scans, updates the scan plans of the one or more remaining diagnostic scans to ensure alignment of the geometries of the remaining diagnostic scans with the geometries of the completed diagnostic scans. This is important because, if the position of the patient **12** changes between two consecutive diagnostic scans, the same voxel in the two diagnostic scans will point to different locations within the patient **12.** Suitably, the scan plans of the remaining diagnostic scans are updated right before performing the corresponding diagnostic scans in case there is additional patient motion.

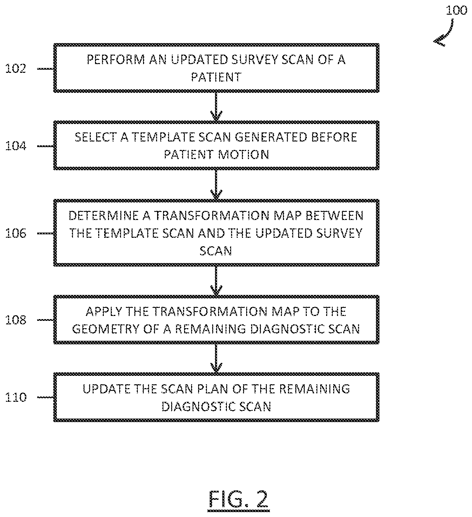

With reference to FIGURE 2, the processor **40** is controlled to perform a method **100** for updating the scan plan of a diagnostic scan after patient motion is detected. The geometric alignment module **58** suitably performs the method **100** for each of the remaining diagnostic scans to update the scan plans of the remaining diagnostic scans. The method **100** includes performing **102** an updated survey scan, such as a fast, three-dimensional (3D) survey scan, of the patient **12** using the data acquisition module **50.** The updated survey scan is generally short, such as less than a minute. Further, the contrast type of the updated survey scan can, but need not, be selected to match the contrast type of the initial survey scan.

A template scan is further selected **104** from the most recently completed diagnostic scan. Further, the contrast type of the template scan can, but need not, match the contrast type of the updated survey scan. For example, the survey scans and the template scan can have different contrast types. Further, the selection is performed automatically.

As an example outside the scope of the invention, the initial survey scan could have been employed. This could be advantageous for registration, discussed hereafter, because the updated survey scan can be chosen to have a similar MR contrast as the initial survey scan. However, this may not lead to optimal alignment in cases where there was some patient motion between the initial survey scan and the most recently completed diagnostic scan.

A registration module **60** of the processor executable instructions is employed to determine **106** a transformation map between the template scan and the updated survey scan within image-space or k-space. Any registration algorithm can be employed. However, a rigid registration algorithm is preferably employed, since no severe anatomical changes occur before and after repositioning and only rigid transformations, such as rotations and translations, can be taken into account for scan planning. Further, rigid registration algorithms are generally faster and more robust than affine or elastic registration algorithms.

Since the quality of the registration depends on the similarity of the template scan and the updated survey scan, and of the quality metrics used in the registration algorithm, the registration algorithm can be adapted to the MR contrast types of the template scan and of the updated survey scan. For example, if the template scan and the updated survey scan have similar MR contrasts, a cross-correlation based registration algorithm can be applied. However, if the template scan and the updated survey scan have different contrast types, a mutual-information based registration algorithm can be applied.

The transformation map is applied **108** to the geometry of the diagnostic scan, and the planning module **56** is employed to update **110** the initially defined scan plan for the diagnostic scan using the updated survey scan and the updated geometry. The updating of the geometry can be automatic or subject to operator supervision. For example, the planning module **56** displays the updated survey scan on the GUI with the updated scan geometry overlaid thereon. An operator of the system **10** can then manipulate the scan geometry using the user input device **48** as desired. Once the desired scan geometry is achieved, the planning module **56** updates the scan plan of the diagnostic scan according to the manipulated scan geometry.

It is to be appreciated that when the remaining diagnostic scans include a plurality of diagnostic scans, the method **100** is performed multiple times, once for each of the remaining diagnostic scans. While each step of the method **100** can be performed for each of the remaining diagnostic scans, in some embodiments, steps are only performed as needed. For example, the selection of the template scan can only be performed once for all of the remaining diagnostic scans. As another example, the updated survey scan can only be performed if there was additional patient motion from the time of generating the last survey scan to the present time. As another example, the transformation map is only determined if a transformation map has not been previously generated with the combination of the selected template scan and the updated survey scan.

Referring back to FIGURE 1, the geometric alignment module **58** can further verify geometric consistency between a reference scan acquired before repositioning and an updated survey scan. The reference scan typically includes one or more low-resolution MR anatomical images with a given MR contrast type, which can be processed to compute a map of a desired quantity, such as receive sensitivity, transmit sensitivity, B0, and so on. The reference scan can, for example, be a receive coil sensitivity scan, as performed during an examinations based on a multi-channel receive coil, or a transmit coil sensitivity scan, as performed during an examination using a multi-channel transmit coil. The reference scan can also, for example, be a B0 or B1 mapping scan.

To verify geometric consistency, Actions **102** to **106** of FIGURE 2 are performed, as described above. The template scan can, for example, be the reference scan. The transformation map is then analyzed to determine the extent of the transformation. Insofar as the extent of the transformation is less than a threshold, the reference scan is registered to the updated survey scan, as described. Otherwise, an operator of the system **10** can be asked to repeat the reference scan using, for example, the GUI or the reference scan can be automatically repeated. To repeat the reference scan, the scan plan of the reference scan is updated to using the updated survey scan and the reference scan is repeated with the updated scan plan.

Alternatively, an updated survey scan is performed, as described above. The reference scan is then registered to the updated survey scan, as described below. The registered reference scan is compared with the updated survey scan. Insofar as the reference scan is not geometrically consistent, an operator of the system **10** can be asked to repeat the reference scan using, for example, the GUI or the reference scan can be automatically repeated.

The geometric alignment **58** module can further register the reference scan to the updated survey scan so that scans acquired after repositioning can use the reference scan. Registering the reference scan to the updated survey scan includes determining a transformation map between the reference scan and the updated survey scan, as described above in FIGURE 2. In determining the transformation map, raw image data (i.e., directly obtained from the k-space data by means of Fourier transform) acquired during the reference scan, or a map computed from the raw image data, is employed. It is, however, preferred to use the raw image data, since the computed map often does not contain anatomical information anymore, and is thus not well suited for registration with the updated survey scan.

A motion module **62** of the processor executable instructions detects patient motion. Motion can be detected manually and/or automatically. As to manual detection, an operator of the system **10** specifies when there is patient motion, for example, using the user input device **48.** As to automatic detection, any number of well-known approaches for detecting motion can be employed. For example, before each full MR scan, an image or map of the patient is generated using a camera **64,** a fast MR scan, or the like. A current image or map is then compared with a reference image or map generated at the beginning of the examination to detect motion. As another example, before each full MR scan, the locations of electromagnetic transponders **66** positioned on the patient are determined. Current locations are then compared with reference locations determined at the beginning of the examination to detect motion. Navigator sequences can also be used to detect patient motion.

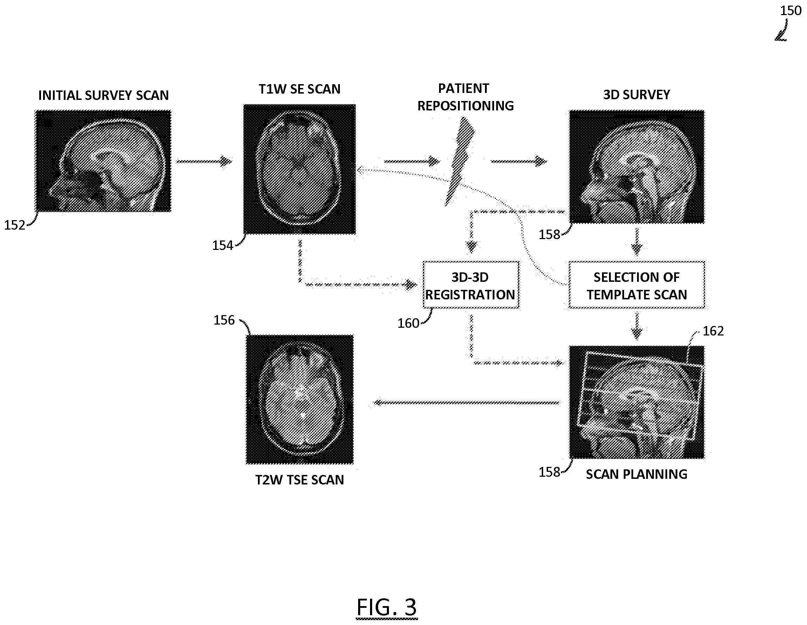

With reference to FIGURE 3, an example **150** of an examination with patient repositioning is provided. The examination begins with an initial survey scan **152** of the patient **12.** Thereafter, a first diagnostic scan **154** is planned using the initial survey scan **152** and then performed. The first diagnostic scan **154** is, for example, a T1 weighted spin echo scan. Sometime between completing the first diagnostic scan **154** and beginning a second diagnostic scan **156,** the patient is repositioned.

To ensure geometric alignment between the first diagnostic scan **154** and the second diagnostic scan **156,** the method **100** of FIGURE 2 is performed. As discussed above, this includes performing an updated survey scan **158,** such as a 3D scan. As can be seen by comparing the initial survey scan **152** and the updated survey scan **158,** the position of the patient **12** has slightly changed (i.e., a translation in the head direction). The first diagnostic scan **154** is then selected as a template scan, and a transformation map between the updated survey scan **158** and the template scan **154** is determined **160** using a registration algorithm, such as a 3D rigid registration algorithm. The transformation map is applied to the scan geometry of the second diagnostic scan **156,** and the updated scan geometry **162** is overlaid on a visualization of the updated survey scan **158.** The updated scan geometry **162** is represented by the updated positions of the slices of the second diagnostic scan **156.** An operator of the system **10** approves the updated scan geometry **162,** and the scan plan of the second diagnostic image **156** is updated with the updated scan geometry **162.** The second diagnostic scan **156** is then performed using the updated scan plan. The second diagnostic scan **156** is, for example, a T2 weighted turbo spin echo scan.

The above described approach to maintaining geometric alignment finds application in any MR clinical applications, including MR-guided interventions and MR-guided radiation therapy. For example, as shown in FIGURE 1, MR is employed to guide a shaft or needle **68** to a target **70** within the patient **12.** Namely, MR images of the shaft or needle **68** can be displayed on, for example, the display device **46,** to the interventionist while they insert the shaft or needle **68.** Additionally, or alternatively, audio and/or visual indications as to how to move the shaft or needle into the planned trajectory can be provided to the interventionist. Other examples of MR-guided interventions where the above described approach can be applied include, but are not limited to, MR-guided high-intensity focused ultrasound, MR-guided laser ablation, MR-guided radiofrequency ablation, MR-guided external beam radiotherapy, MR-guided brachytherapy, and so on. In the cases of MR guidance, the operator may have repositioned the patient intentionally during the intervention. Also, in those cases, the template scan used for the registration may have been acquired prior to the intervention during an examination used for planning the intervention. Further, images acquired from diagnostic scans advantageously maintain geometric alignment without exhibiting partial volume effects when there is patient motion between the diagnostic scans. During interventions, there is generally no time to perform post-processing steps, so it is beneficial to acquire the images directly in the desired geometry.

As used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet/Intranet server from which the stored instructions may be retrieved via the Internet/Intranet or a local area network; or so forth. Further, as used herein, a processor includes one or more of a microprocessor, a microcontroller, a graphic processing unit (GPU), an application-specific integrated circuit (ASIC), an FPGA, and the like; a controller includes: (1) a processor and a memory, the processor executing computer executable instructions on the memory embodying the functionality of the controller; or (2) analog and/or digital hardware; a user input device includes one or more of a mouse, a keyboard, a touch screen display, one or more buttons, one or more switches, one or more toggles, voice recognition engines, and the like; a database includes one or more memories; and a display device includes one or more of a LCD display, an LED display, a plasma display, a projection display, a touch screen display, and the like.

The invention has been described with reference to the preferred embodiments. Modifications and alterations may occur to others upon reading and understanding the preceding detailed description. It is intended that the invention be construed as including all such modifications and alterations insofar as they come within the scope of the appended claims.

## Claims

1. A magnetic resonance (MR) system **(10)** for maintaining geometric alignment of diagnostic scans during an examination of a patient **(12),** said system **(10)** comprising:
at least one processor **(40)** programmed to:
in response to repositioning of the patient **(12)** during the examination:
perform an updated survey scan of the patient **(12);**
select the diagnostic scan most recently completed during the examination as a template scan;
determine a transformation map between the template scan and the updated survey scan using a registration algorithm;
wherein the registration algorithm is chosen according to the MR contrast types of the template scan and the survey scan, whereby if the template scan and the updated survey scan have similar MR contrast types, a cross-correlation based registration algorithm is applied, and if the template scan and the updated survey scan have different contrast types, a mutual-information based registration algorithm is applied;
apply the transformation map to an initially defined scan geometry of a remaining diagnostic scan of the examination;
generate a scan plan for the remaining diagnostic scan
using the updated scan geometry; and,
perform the remaining diagnostic scan according to the scan plan.

2. The system **(10)** according to claim 1, further including:
an MR scanner **(14)** controlled to perform scans of the examination, including the updated survey scan, the template scan, and the remaining diagnostic scan.

3. The system **(10)** according to either one of claims 1 and 2, wherein the updated survey scan is a fast, three-dimensional survey scan.

4. The system **(10)** according to any one of claims 1-3, wherein the transformation map is determined using a rigid registration algorithm.

5. The system **(10)** according to any one of claims 1-4, wherein the diagnostic scans are acquired during an MR-guided intervention.

6. The system **(10)** according to any one of claims 1-5, wherein the at least one processor **(40)** is further programmed to:
perform an initial survey scan, wherein the template scan is one of: (1) the initial survey scan; and (2) a diagnostic scan planned using the initial survey scan.

7. The system **(10)** according to any one of claims 1 -6, wherein an MR contrast type of the updated survey scan is different than an MR contrast type of the template scan.

8. The system **(10)** according to any one of claims 1-7, wherein generating the scan plan includes:
displaying the updated survey scan with the updated scan geometry overlaid on the updated survey scan; and,
receiving acceptance of the updated scan geometry or modifications to the updated scan geometry.

9. The system **(10)** according to claim 8, wherein the updated scan geometry is overlaid on the updated survey scan by displaying positions of corresponding slices.

10. The system **(10)** according to any one of claims 1-9, wherein the at least one processor **(40)** is further programmed to:
automatically detect repositioning or strong motion of the patient.

11. A magnetic resonance (MR) method **(100)** for maintaining geometric alignment of diagnostic scans during an examination of a patient **(12),** said method **(100)** comprising:
in response to repositioning of the patient **(12)** during the examination:
performing **(102)** by at least one processor **(40)** an updated survey scan of the patient **(12);**
selecting **(104)** by the at least one processor **(40)** the diagnostic scan most recently completed during the examination as a template scan;
determining **(106)** by the at least one processor **(40)** a transformation map between the template scan and the updated survey scan using a registration algorithm;
wherein the registration algorithm is chosen according to the MR contrast types of the template scan and the survey scan, whereby if the template scan and the updated survey scan have similar MR contrast types, a cross-correlation based registration algorithm is applied, and if the template scan and the updated survey scan have different contrast types, a mutual-information based registration algorithm is applied;
applying **(108)** by the at least one processor **(40)** the transformation map to a scan geometry of a remaining diagnostic scan of the examination;
generating **(110)** by the at least one processor **(40)** a scan plan for the remaining diagnostic scan using the updated scan geometry; and,
performing the remaining diagnostic scan according to the scan plan, **characterized in that** the templet scan is automatically selected, wherein the scan selected as a template scan by the at least one processor **(40)** is the diagnostic scan most recently completed during the examination.

12. The method **(100)** according to claim 11, further including:
performing scans of the examination, including the updated survey scan, the template scan, and the remaining diagnostic scan, using an MR scanner **(14).**

13. The method **(100)** according to either one of claims 1 1 and 12, wherein the updated survey scan is a fast, three-dimensional survey scan.

14. The method **(100)** according to any one of claims 1 1-13, wherein the transformation map is determined using a rigid registration algorithm.

15. The method **(100)** according to any one of claims 1 1-14, wherein the diagnostic scans are acquired during an MR-guided intervention.

16. The method **(100)** according to any one of claims 11 -15, furthering including:
performing an initial survey scan, wherein the template scan is one of: (1) the initial survey scan; and (2) a diagnostic scan planned using the initial survey scan;
wherein an MR contrast type of the updated survey scan is different than an MR contrast type of the template scan.

17. The method **(100)** according to any one of claims 1 1-16, wherein generating **(110)** the scan plan includes:
displaying the updated survey scan with the updated scan geometry overlaid on the updated survey scan; and,
receiving acceptance of the updated scan geometry or modifications to the updated scan geometry.

18. The method **(100)** according to any one of claims 11-17, wherein further including:
automatically detecting repositioning or strong motion of the patient **(12).**

## Patentansprüche

1. Magnetresonanz- (MR) System (10) zur Bewahrung der geometrischen Ausrichtung von Diagnosescans während einer Untersuchung eines Patienten (12), wobei das genannte System (10) Folgendes umfasst:
mindestens einen Prozessor (40), der programmiert ist zum:
in Reaktion auf die Neupositionierung des Patienten (12) während der Untersuchung:
Durchführen eines aktualisierten Übersichtsscans des Patienten (12);
Auswählen des zuletzt während der Untersuchung durchgeführten Diagnosescans als einen Vorlagescan;
Festlegen einer Transformationskarte zwischen dem Vorlagescan und dem aktualisierten Übersichtsscan mithilfe eines Registrierungsalgorithmus;
wobei der Registrierungsalgorithmus entsprechend den MR-Kontrasttypen des Vorlagescans und des Übersichtsscans gewählt wird, so dass, wenn der Vorlagescan und der aktualisierte Übersichtsscan ähnliche MR-Kontrasttypen aufweisen, eine Kreuzkorrelation basierend auf dem Registrierungsalgorithmus angewendet wird, und wenn der Vorlagescan und der aktualisierte Übersichtsscan unterschiedliche Kontrasttypen aufweisen, eine gegenseitige Information basierend auf dem Registrierungsalgorithmus angewendet wird;
Anwenden der Transformationskarte auf eine anfangs definierte Scan-Geometrie eines verbleibenden Diagnosescans der Untersuchung;
Erzeugen eines Scan-Plans für den verbleibenden Diagnosescan mithilfe der aktualisierten Scan-Geometrie; und
Durchführen des verbleibenden Diagnosescans entsprechend dem Scan-Plan.

2. System (10) nach Anspruch 1, das weiterhin Folgendes umfasst:
einen MR-Scanner (14), der gesteuert wird, um Scans der Untersuchung durchzuführen, einschließlich des aktualisierten Übersichtsscans, des Vorlagescans und des verbleibenden Diagnosescans.

3. System (10) nach einem der Ansprüche 1 und 2, wobei der aktualisierte Übersichtsscan ein schneller, dreidimensionaler Übersichtsscan ist.

4. System (10) nach einem der Ansprüche 1 bis 3, wobei die Transformationskarte mithilfe eines starren Registrierungsalgorithmus festgelegt wird.

5. System (10) nach einem der Ansprüche 1 bis 4, wobei die Diagnosescans während eines MR-geführten Eingriffs erfasst werden.

6. System (10) nach einem der Ansprüche 1 bis 5, wobei der mindestens eine Prozessor (40) weiterhin programmiert ist zum:
Durchführen eines anfänglichen Übersichtsscans, wobei der Vorlagescan eines ist von: (1) dem anfänglichen Übersichtsscan und (2) einem Diagnosescan, der mithilfe des anfänglichen Übersichtsscans geplant wurde.

7. System (10) nach einem der Ansprüche 1 bis 6, wobei ein MR-Kontrasttyp des aktualisierten Übersichtsscans sich von einem MR-Kontrasttyp des Vorlagescans unterscheidet.

8. System (10) nach einem der Ansprüche 1 bis 7, wobei das Erzeugen des Scan-Plans Folgendes umfasst:
Anzeigen des aktualisierten Übersichtsscans mit überlagerter aktualisierter Scan-Geometrie über dem aktualisierten Übersichtsscan; und
Empfangen der Akzeptanz der aktualisierten Scan-Geometrie oder von Modifikationen an der aktualisierten Scan-Geometrie.

9. System (10) nach Anspruch 8, wobei die aktualisierte Scan-Geometrie dem aktualisierten Übersichtsscan überlagert wird, indem Positionen von übereinstimmenden Schichten angezeigt werden.

10. System (10) nach einem der Ansprüche 1 bis 9, wobei der mindestens eine Prozessor (40) weiterhin programmiert ist zum:
automatischen Erkennen der Neupositionierung oder einer starken Bewegung des Patienten.

11. Magnetresonanz- (MR) Verfahren (100) zur Bewahrung der geometrischen Ausrichtung von Diagnosescans während einer Untersuchung eines Patienten (12), wobei das genannte Verfahren (100) Folgendes umfasst:
in Reaktion auf die Neupositionierung des Patienten (12) während der Untersuchung:
Durchführen (102) eines aktualisierten Übersichtsscans des Patienten (12) durch mindestens einen Prozessor (40);
Auswählen (104), durch den mindestens einen Prozessor (40), des zuletzt während der Untersuchung durchgeführten Diagnosescans als einen Vorlagescan;
Festlegen (106), durch den mindestens einen Prozessor (40), einer Transformationskarte zwischen dem Vorlagescan und dem aktualisierten Übersichtsscan mithilfe eines Registrierungsalgorithmus;
wobei der Registrierungsalgorithmus entsprechend den MR-Kontrasttypen des Vorlagescans und des Übersichtsscans gewählt wird, so dass, wenn der Vorlagescan und der aktualisierte Übersichtsscan ähnliche MR-Kontrasttypen aufweisen, eine Kreuzkorrelation basierend auf dem Registrierungsalgorithmus angewendet wird, und wenn der Vorlagescan und der aktualisierte Übersichtsscan unterschiedliche Kontrasttypen aufweisen, eine gegenseitige Information basierend auf dem Registrierungsalgorithmus angewendet wird;
Anwenden (108), durch den mindestens einen Prozessor (40), der Transformationskarte auf eine Scan-Geometrie eines verbleibenden Diagnosescans der Untersuchung;
Erzeugen (110), durch den mindestens einen Prozessor (40), eines Scan-Plans für den verbleibenden Diagnosescan mithilfe der aktualisierten Scan-Geometrie; und
Durchführen des verbleibenden Diagnosescans entsprechend dem Scan-Plan, **dadurch gekennzeichnet, dass** der Vorlagescan automatisch gewählt wird, wobei der durch den mindestens einen Prozessor (40) als Vorlagescan gewählte Scan der zuletzt während der Untersuchung durchgeführte Diagnosescan ist.

12. Verfahren (100) nach Anspruch 11, das weiterhin Folgendes umfasst:
Durchführen von Scans der Untersuchung, einschließlich des aktualisierten Übersichtsscans, des Vorlagescans und des verbleibenden Diagnosescans, mithilfe eines MR-Scanners (14).

13. Verfahren (100) nach einem der Ansprüche 11 und 12, wobei der aktualisierte Übersichtsscan ein schneller, dreidimensionaler Übersichtsscan ist.

14. Verfahren (100) nach einem der Ansprüche 11 bis 13, wobei die Transformationskarte mithilfe eines starren Registrierungsalgorithmus festgelegt wird.

15. Verfahren (100) nach einem der Ansprüche 11 bis 14, wobei die Diagnosescans während eines MR-geführten Eingriffs erfasst werden.

16. Verfahren (100) nach einem der Ansprüche 11 bis 15, das weiterhin Folgendes umfasst:
Durchführen eines anfänglichen Übersichtsscans, wobei der Vorlagescan eines ist von: (1) dem anfänglichen Übersichtsscan und (2) einem Diagnosescan, der mithilfe des anfänglichen Übersichtsscans geplant wurde;
wobei ein MR-Kontrasttyp des aktualisierten Übersichtsscans sich von einem MR-Kontrasttyp des Vorlagescan unterscheidet.

17. Verfahren (100) nach einem der Ansprüche 11 bis 16, wobei das Erzeugen (110) des Scan-Plans Folgendes umfasst:
Anzeigen des aktualisierten Übersichtsscans mit überlagerter aktualisierter Scan-Geometrie über dem aktualisierten Übersichtsscan; und
Empfangen der Akzeptanz der aktualisierten Scan-Geometrie oder von Modifikationen an der aktualisierten Scan-Geometrie.

18. Verfahren (100) nach einem der Ansprüche 11 bis 17, das weiterhin Folgendes umfasst:
automatisches Erkennen der Neupositionierung oder einer starken Bewegung des Patienten (12).

## Revendications

1. Système à résonance magnétique (MR) (10) pour maintenir un alignement géométrique de balayages de diagnostic durant un examen d'un patient (12), ledit système (10) comprenant :
au moins un processeur (40) programmé pour :
en réponse au repositionnement du patient (12) durant l'examen :
la réalisation d'un balayage d'inspection actualisé du patient (12) ;
la sélection du balayage de diagnostic achevé le plus récemment durant l'examen en tant que balayage modèle ;
la détermination d'une carte de transformation entre le balayage modèle et le balayage d'inspection actualisé en utilisant un algorithme d'enregistrement ;
dans lequel l'algorithme d'enregistrement est choisi selon les types de contraste de MR du balayage modèle et du balayage d'inspection, moyennant quoi, si le balayage modèle et le balayage d'inspection actualisé possèdent des types de contraste de MR similaires, un algorithme d'enregistrement à base de corrélation croisée est appliqué, et, si le balayage modèle et le balayage d'inspection actualisé possèdent des types de contraste différents, un algorithme d'enregistrement à base d'informations mutuelles est appliqué ;
l'application de la carte de transformation sur une géométrie de balayage définie initialement d'un autre balayage de diagnostic de l'examen ;
la génération d'un plan de balayage pour l'autre balayage de diagnostic en utilisant la géométrie de balayage actualisée ; et,
la réalisation de l'autre balayage de diagnostic selon le plan de balayage.

2. Système (10) selon la revendication 1, incluant en outre :
un dispositif de balayage à MR (14) commandé pour réaliser des balayages de l'examen, incluant le balayage d'inspection actualisé, le balayage modèle, et l'autre balayage de diagnostic.

3. Système (10) selon l'une ou l'autre des revendications 1 et 2, dans lequel le balayage d'inspection actualisé est un balayage d'inspection tridimensionnel rapide.

4. Système (10) selon l'une quelconque des revendications 1 à 3, dans lequel la carte de transformation est déterminée en utilisant un algorithme d'enregistrement rigide.

5. Système (10) selon l'une quelconque des revendications 1 à 4, dans lequel les balayages de diagnostic sont acquis durant une intervention guidée par MR.

6. Système (10) selon l'une quelconque des revendications 1 à 5, dans lequel l'au moins un processeur (40) est en outre programmé pour :
réaliser un balayage d'inspection initial, dans lequel le balayage modèle est l'un parmi : (1) le balayage d'inspection initial ; et (2) un balayage de diagnostic planifié en utilisant le balayage d'inspection initial.

7. Système (10) selon l'une quelconque des revendications 1 à 6, dans lequel un type de contraste de MR du balayage d'inspection actualisé est différent d'un type de contraste de MR du balayage modèle.

8. Système (10) selon l'une quelconque des revendications 1 à 7, dans lequel la génération du plan de balayage inclut :
l'affichage du balayage d'inspection actualisé avec la géométrie de balayage actualisée superposée sur le balayage d'inspection actualisé ; et,
la réception de l'acceptation de la géométrie de balayage actualisée ou de modifications de la géométrie de balayage actualisée.

9. Système (10) selon la revendication 8, dans lequel la géométrie de balayage actualisée est superposée sur le balayage d'inspection actualisé en affichant des positions de tranches correspondantes.

10. Système (10) selon l'une quelconque des revendications 1 à 9, dans lequel l'au moins un processeur (40) est en outre programmé pour :
la détection automatique de repositionnement ou de fort mouvement du patient.

11. Procédé à résonance magnétique (MR) (100) pour maintenir un alignement géométrique de balayages de diagnostic durant un examen d'un patient (12), ledit procédé (100) comprenant :
en réponse à un repositionnement du patient (12) durant l'examen :
la réalisation (102), par au moins un processeur (40), d'un balayage d'inspection actualisé du patient (12) ;
la sélection (104), par l'au moins un processeur (40), du balayage de diagnostic achevé le plus récemment durant l'examen en tant que balayage modèle ;
la détermination (106), par l'au moins un processeur (40), d'une carte de transformation entre le balayage modèle et le balayage d'inspection actualisé en utilisant un algorithme d'enregistrement ;
dans lequel l'algorithme d'enregistrement est choisi selon les types de contraste de MR du balayage modèle et du balayage d'inspection, moyennant quoi, si le balayage modèle et le balayage d'inspection actualisé possèdent des types de contraste de MR similaires, un algorithme d'enregistrement à base de corrélation croisée est appliqué, et, si le balayage modèle et le balayage d'inspection actualisé possèdent des types de contraste différents, un algorithme d'enregistrement à base d'informations mutuelles est appliqué ;
l'application (108), par l'au moins un processeur (40), de la carte de transformation sur une géométrie de balayage d'un autre balayage de diagnostic de l'examen ;
la génération (110), par l'au moins un processeur (40), d'un plan de balayage pour l'autre balayage de diagnostic en utilisant la géométrie de balayage actualisée ; et,
la réalisation de l'autre balayage de diagnostic selon le plan de balayage, **caractérisé en ce que** le balayage modèle est automatiquement sélectionné, dans lequel le balayage sélectionné en tant que balayage modèle par l'au moins un processeur (40) est le balayage de diagnostic achevé le plus récemment durant l'examen.

12. Procédé (100) selon la revendication 11, incluant en outre :
la réalisation de balayages de l'examen, incluant le balayage d'inspection actualisé, le balayage modèle, et l'autre balayage de diagnostic, en utilisant un dispositif de balayage à MR (14).

13. Procédé (100) selon l'une ou l'autre des revendications 11 et 12, dans lequel le balayage d'inspection actualisé est un balayage d'inspection tridimensionnel rapide.

14. Procédé (100) selon l'une quelconque des revendications 11 à 13, dans lequel la carte de transformation est déterminée en utilisant un algorithme d'enregistrement rigide.

15. Procédé (100) selon l'une quelconque des revendications 11 à 14, dans lequel les balayages de diagnostic sont acquis durant une intervention guidée par MR.

16. Procédé (100) selon l'une quelconque des revendications 11 à 15, incluant en outre :
la réalisation d'un balayage d'inspection initial, dans lequel le balayage modèle est l'un parmi : (1) le balayage d'inspection initial ; et (2) un balayage de diagnostic planifié en utilisant le balayage d'inspection initial ;
dans lequel un type de contraste de MR du balayage d'inspection actualisé est différent d'un type de contraste de MR du balayage modèle.

17. Procédé (100) selon l'une quelconque des revendications 11 à 16, dans lequel la génération (110) du plan de balayage inclut :
l'affichage du balayage d'inspection actualisé avec la géométrie de balayage actualisée superposée sur le balayage d'inspection actualisé ; et,
la réception d'acceptation de la géométrie de balayage actualisée ou de modifications de la géométrie de balayage actualisée.

18. Procédé (100) selon l'une quelconque des revendications 11 à 17, incluant en outre :
la détection automatique de repositionnement ou de fort mouvement du patient (12).
